# FASCICULE DE BREVET EUROPEEN

(11) **EP 2 257 244 B1**
(45) Date de publication et mention de la délivrance du brevet: **29.07.2015**
(21) Numéro de dépôt: 09733982.4
(22) Date de dépôt: 27.03.2009
(51) Int. Cl.: A61F 2/34, A61F 2/46

(54) **ÉLÉMENT COTYLOÏDIEN DE PROTHÈSE DE HANCHE, ET PROTHÈSE TOTALE DE HANCHE LE COMPORTANT**
GELENKPFANNENELEMENT FÜR EINE HÜFTPROTHESE UND HÜFTTOTALENDOPROTHESE DAMIT
COTYLOID ELEMENT OF A HIP PROSTHESIS, AND TOTAL HIP PROSTHESIS COMPRISING SAME

(30) Priorité: 28.03.2008 FR 0801719
(43) Date de publication de la demande: 08.12.2010
(73) Titulaire: T.O., 69002 Lyon (FR)
(72) Inventeur: THEILLEZ, Boris, F-26300 Bourg de Peage (FR); CHANZY, Nicolas, F-92100 Boulogne Billancourt (FR); FARISON, Frédéric, F-42400 St. Chamond (FR); ADAM, Philippe, F-67117 Quatzenheim (FR); PHILIPPOT, Rémi, F-42000 Saint Etienne (FR); FAVRE, Eric, F-73000 Chambéry (FR); LIMOZIN, Rodolphe, F-12000 Rodez (FR); PROVOST, Gilles, F-01240 Certines (FR); DOYCINOVICH, Serge, F-01000 Bourg en Bresse (FR); CAMILLERI, Jean-Philippe, F-69008 Lyon (FR)
(74) Mandataire: Schmidt, Martin Peter
(86) Numéro de dépôt international: PCT/FR2009/000341
(87) Numéro de publication internationale: WO 2009/130406

(56) Documents cités:
- DE-U- 9 402 828
- FR-A- 2 554 342
- FR-A- 2 583 634
- FR-A- 2 585 946
- US-A- 5 458 649

## Description

### Domaine de l'invention

L'invention concerne les prothèses de hanche, et plus précisément les éléments cotyloïdiens des prothèses de hanche destinés à être implantés sans ciment.

### Etat de la technique

Les prothèses de hanche sont des implants couramment utilisés en chirurgie humaine pour traiter les fractures de l'extrémité supérieure du fémur, ainsi que les affections rhumatismales évoluées de la hanche. Elles peuvent être uniquement fémoraux, c'est-à-dire ne comporter qu'un seul élément destiné à remplacer l'extrémité supérieure du fémur et pourvu à son extrémité d'une tête sphérique destinée à s'insérer dans la cavité cotyloïdienne du patient. Elles peuvent être totales, c'est-à-dire comporter, en plus de l'élément fémoral, un cotyle artificiel que le chirurgien implante dans l'os iliaque du patient, à la place de la cavité naturelle du patient.

Dans les prothèses totales de hanche, l'élément fémoral comporte de manière habituelle :
- Une tige de forme sensiblement rectiligne ancrée dans le fémur et finissant par une extrémité distale conique (col) pour insertion de la tête sphérique de diamètre variable.
- Et une tête sphérique placée à l'extrémité du col prothétique.

L'élément cotyloïdien comporte de manière habituelle une partie fixée dans la cavité naturelle de l'os iliaque. Cette partie fixée dans l'os est pourvue d'une surface de glissement ayant la forme d'une cavité hémisphérique.

Selon les types de prothèse, l'élément cotyloïdien peut être composé d'une seule pièce en métal, en céramique ou polyéthylène. Toutefois, pour permettre la fixation sans ciment du cotyle dans l'os iliaque, beaucoup de modèles, en particulier ceux dits « à double mobilité » se composent des éléments suivants :
- Une cupule métallique, généralement sous la forme d'une calotte, fixée dans l'os,
- Un insert en polyéthylène, placé dans la cupule et dans lequel vient s'articuler la tête prothétique sphérique de l'élément fémoral.

Les principaux problèmes rencontrés dans la pratique chirurgicale sont d'une part l'usure des surfaces entre la tête fémorale et la pièce cotyloïdienne, liée au frottement sous charge, et d'autre part la fixation de la cupule métallique dans l'os.

Plusieurs modes de fixation de la cupule métallique sont actuellement utilisés, notamment le scellement (a), l'impaction (b), l'expansion (c), le vissage (d), détaillés ci-après.
a) Le scellement consiste à placer entre l'os et la cupule un ciment polymérisable à base acrylique qui s'ancre dans l'os et assure la fixation primaire et secondaire de la cupule. Cependant, un descellement peut apparaître entre l'os et la cupule à plus ou moins long terme notamment à cause des caractéristiques de l'os et du ciment.
b) L'impaction consiste à insérer la cupule métallique dans le cotyle osseux préparé par des fraises hémisphériques calibrant l'os à un diamètre inférieur au diamètre de la cupule métallique. Un revêtement et des aspérités de surfaces situées en périphérie de la cupule améliorent la fixation primaire et augmente la repousse osseuse. Cette tenue mécanique dépend de la préparation osseuse mais aussi et surtout de la qualité de l'os. Beaucoup de modèles proposent des fixations complémentaires, par exemple de type plots ou vis, mis en place dans des trous aménagés dans la cupule métallique.
   Cette technique comporte en particulier les inconvénients suivants : une micromobilité est possible entre les vis ou les plots, une mauvaise impaction des plots ou des vis augmente 'usure.
c) L'expansion consiste à utiliser une cupule métallique conçue de manière à être ouverte dans la cavité osseuse préalablement préparées comme en b). Il existe deux types de cupule à expansion :
   a. Les cupules pourvues de pétales répartis symétriquement sur la périphérie de la cupule et retenus par une bague qui est ôtée lors de l'insertion de la cupule pour assurer l'expansion.
   b. Un insert vissé à l'intérieur de la cupule métallique et dont le vissage assure l'expansion.
      Les cupules à expansion ont en particulier les inconvénients suivants : l'insert en plastique doit encaisser tous les efforts et ne pas se déformer pour assurer durablement l'expansion ; dans le cas de la cupule pourvues de pétales, des fentes augmentent le contact os/partie plastique du fait du phénomène de fluage ce qui favorise l'ostéolyse.
   d) Dans les cupules vissées, une cupule métallique présentant un filetage sur sa surface externe est directement vissée dans l'os iliaque. La partie externe de la cupule peut être recouverte de matériaux favorisant la repousse osseuse.

Ce type de cotyle est connu et décrit dans de nombreux documents de l'état de la technique. Par exemple, le brevet FR 2 622 432 concerne un anneau de cotyle vissable pour prothèse de hanche. L'anneau décrit dans ce brevet est de forme tronconique, il est pourvu d'un filetage auto-taraudant. La demande de brevet FR 2 639 822 décrit un cotyle sensiblement hémisphérique pourvu d'un filetage externe. Dans ce document l'ancrage de la prothèse dans l'os est facilité par la présence de languettes déformables élastiquement pour être repoussées après vissage du cotyle dans l'os par des moyens rapportés aptes à coopérer avec au moins une languette sélectionnée. Le brevet US 6,146,425 concerne un cotyle pourvu d'un filetage externe auto-taraudant dont les arêtes possède une orientation angulaire particulière et un pas variable pour un vissage facilité et un meilleur ancrage dans l'os iliaque.

Un tel dispositif est également connu du brevet français 2 585 946, correspondant au préambule de la revendication 1. Ce brevet décrit un composant cotyloïdien comprenant une calotte hémisphérique en titane possédant sur sa surface externe un filetage auto-taraudant à double filet interrompu.

Les inconvénients des cotyles vissés tels que décrits dans les documents de l'état de la technique précédents sont les suivants: un mauvais positionnement lors de la mise en place augmente le taux de luxation, favorise l'usure, nuit à l'ostéo-intégration. De plus, la reprise de la prothèse est mal aisée du fait de l'existence du filetage.

La présente invention consiste à pallier ces inconvénients particuliers, pour conduire aux avantages suivants :
- Une tenue mécanique optimale quelle que soit la qualité osseuse,
- Une mise en place précise et aisée,
- Une usure réduite au niveau de l'articulation,
- La possibilité de mettre un insert polyéthylène mobile ou une tête articulée métallique de gros diamètre directement dans la cupule.

### Objet de l'invention

Un premier objet de la présente invention est un composant cotyloïdien de prothèse de hanche, ledit composant cotyloïdien étant creux et en forme de cupule dont la partie externe comporte un filetage pour permettre la fixation dudit composant cotyloïdien dans la cavité cotyloïde de l'os iliaque convenablement préparée, ledit filetage comportant un double filet (20, 21) auto-taraudant interrompu, ledit composant cotyloïdien étant pourvu d'un pôle supérieur effacé (1), et ledit composant cotyloïdien étant pourvu sur sa face externe (10) d'un revêtement favorisant l'ostéo-intégration (typiquement un revêtement de titane poreux associé à un revêtement d'hydroxyapatite de calcium), et ledit composant cotyloïden étant pourvu d'une surface interne concave sensiblement hémisphérique polie (11), et ledit composant cotyloïdien étant caractérisé en ce que
- le pas des filetages (20, 21) est décroissant du pôle supérieur (1) vers la périphérie équatoriale (3) du composant cotyloïdien,
- les épaisseurs des filets (20, 21) sont croissantes du pôle supérieur (1) du composant cotyloïdien vers sa périphérie (3),
- l'arête des filets (20, 21) est coupante du côté du pôle (1) du composant cotyloïdien et arrondie ou sensiblement trapézoïdale du côté de la périphérie équatoriale (3) du composant cotyloïdien, le profil de ladite arête variant de façon continue et régulière depuis le pôle (1) vers la périphérie équatoriale (3).

Avantageusement, le revêtement favorisant l'ostéointé-gration est absent sur une couronne de hauteur h comprise entre 2 et 5 mm sur la périphérie équatoriale du cotyle, et préférentiellement sur une hauteur h comprise entre 3 et 4 mm.

L'autre objet qui ne tombe pas sous l'objectif de la présente invention est une platine permettant la pose par vissage et la dépose par dévissage du cotyle selon l'invention dans l'os iliaque par préhension à l'aide des encoches de vissage / dévissage (4), caractérisée en ce que la platine comporte des protubérances (9) destinées à coopérer avec les encoches (4) dudit composant cotyloïdien pour permettre la préhension et le vissage ou le dévissage du composant cotyloïdien.

Un dernier objet de l'invention est une prothèse totale de hanche comprenant un composant cotyloidien selon l'invention.

### Description des figures

Les figures la, 1b et 1c représentent respectivement une vue de dessus, une vue de profil et une coupe transversale d'un cotyle selon l'invention.
La figure 2 représente une vue agrandie en coupe du filetage du cotyle selon l'invention. La figure 2a représente une vue en coupe agrandie de la partie entrant la première en contact avec l'os dans le sens du vissage.
La figure 2b représente une vue en coupe agrandie de la partie entrant la dernière en contact avec l'os dans le sens du vissage.
Les figures 3a, 3b, 3c, 3d représentent respectivement une vue de face, une vue en coupe selon la ligne AA, une vue de dessous et une vue de profil d'une platine de serrage du cotyle.

### Liste des repères :

1. Pôle supérieur
20, 21. Filetage double
3. Périphérie équatoriale du composant cotyloïdien
4. Encoches de vissage et dévissage
5. Hauteur h sans revêtement favorisant l'ostéo-intégration 6, 61, 62. Parties de filets situées près du pôle supérieur 7, 71, 72. Parties de filets situées près de la périphérie équatoriale
8. Face d'appui de la platine
9. Protubérances
10. Face externe du cotyle
11. Face interne du cotyle
12. Interruption du filetage

### Description de l'invention

Un premier objet de l'invention est un élément cotyloïdien (appelé cotyle) artificiel creux en forme de cupule.

Le cotyle selon l'invention est de préférence réalisé en métal ou alliage métallique tel que des stellites (alliages chrome-cobalt) ou des aciers inoxydables.

La partie externe du cotyle selon l'invention comporte un filetage pour permettre sa fixation dans l'os iliaque. Ce filetage est auto-taraudant

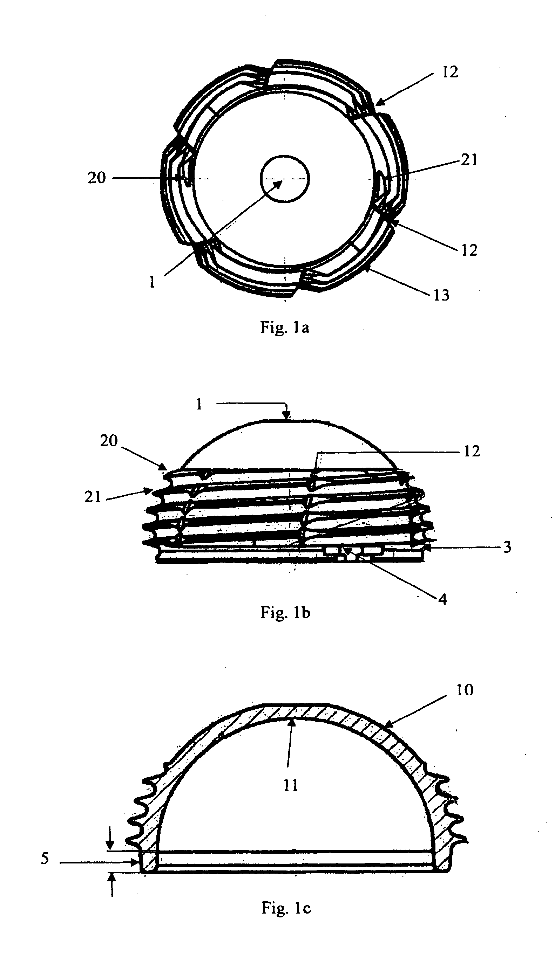

Le filetage du cotyle selon l'invention est un filetage à double filet **20, 21,** tel que représenté sur les figures 1a, 1b, 1c. Le principal avantage d'un tel filetage à double filet **20, 21** est qu'il permet d'augmenter le pas de filetage et par conséquent le nombre de tours de vissage est inférieur au nombre classiquement nécessaire pour les cotyles vissés de l'état de la technique, ce qui permet une fixation rapide du cotyle dans l'os. Le cotyle selon l'invention permet en outre une fixation précise du cotyle dans l'os, grâce au fait que le double filetage possèdent des filets **20, 21** commençant à 180° l'un de l'autre sur le pôle 1 du cotyle tel que représenté sur la figure la, ce qui facilite le positionnement du cotyle de manière précise au début du vissage.

Les filets **20, 21** sont interrompus par des parties non filetées **12,** de manière à ce que l'os puisse être évacué au cours du taraudage causé par la mise en place du cotyle, et à ce que l'os soit attaqué de nouveau par un bord coupant du filetage. Les parties non filetées 12 sont avantageusement au nombre de 6, régulièrement espacées sur la périphérie du cotyle tel que cela est représenté sur les figures 1a et 1b. Elles peuvent toutefois être plus ou moins nombreuses sans que cela change la portée de l'invention. Avantageusement, les parties non filetés correspondent au plus à 25% du filetage, mesuré sur le diamètre externe du filetage.

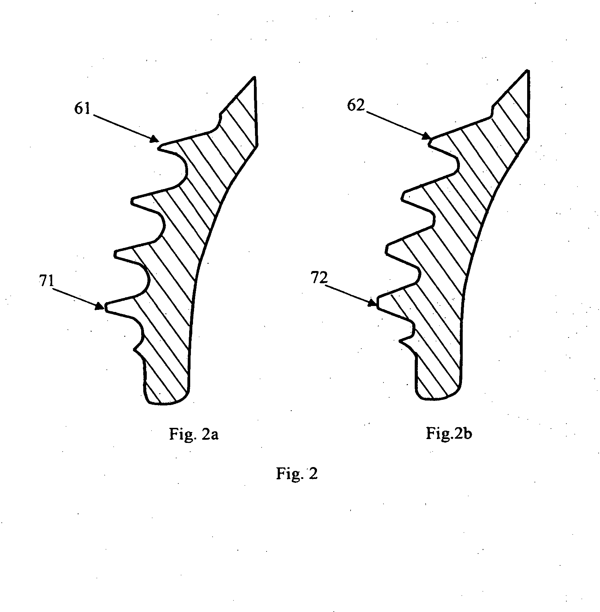

D'autre part, les filets ne sont pas d'épaisseur constante, mais d'épaisseur croissante dans le sens du vissage, depuis le pôle supérieur **1** vers la périphérie équatoriale **3** du cotyle. De plus, ainsi que cela est représenté sur la figure 2, les parties de filets **6** situées près du pôle supérieur **1** possèdent des arêtes vives coupantes de forme sensiblement triangulaire, les parties de filets **7** situées près de la périphérie équatoriale **3** possèdent des arêtes de section arrondie ou trapézoïdale. L'épaisseur des filets est également croissante dans le sens du vissage dans chacune des parties filetées **13** située entre les parties non filetées **12,** ainsi que représentée sur les figures 2a et 2b. La figure 2a représente une vue en coupe agrandie de la partie entrant la première en contact avec l'os dans le sens du vissage. La figure 2b représente une vue en coupe agrandie de la partie entrant la dernière en contact avec l'os dans le sens du vissage.

Le fait d'avoir un filet mince à arête vive coupante du côté du pôle **1,** c'est-à-dire sur la partie du filetage qui entre en contact avec l'os dès le début du vissage du cotyle, et également une partie plus coupante du côté de chaque partie filetée **13** entrant le premier en contact avec l'os dans le sens du vissage permet un auto-taraudage efficace de l'os par le cotyle selon l'invention. Le fait d'avoir un filet à arête plus épaisse du côté de l'équateur, c'est-à-dire en fin de vissage, et également une partie moins coupante du côté de chaque partie filetée **13** entrant le dernier en contact avec l'os dans le sens du vissage permet une meilleure fixation du cotyle selon l'invention dans l'os car de cette manière les filets ont une surface de contact avec l'os importante, et la forme moins coupante des filets génère moins de contraintes mécaniques dans l'os.

Le pas des filetages **20, 21** est décroissant du pôle supérieur **1** vers la périphérie équatoriale **3** du composant cotyloïdien. Le pas des filets **20, 21** est compris entre 2 et 5 mm, et de préférence entre 3 et 4 mm.

Le cotyle selon l'invention, généralement de forme approximativement hémisphérique, est en outre pourvu d'un pôle supérieur effacé 1, représenté sur les figures la, 1b et 1c, pour éviter les problèmes d'hyperpression dans la pose du cotyle dans la cavité cotyloïdienne de l'os iliaque.

Le cotyle selon l'invention est de plus pourvu sur sa face externe 10 d'un revêtement favorisant l'ostéo-intégration, tel qu'une revêtement de titane poreux associé à un revêtement d'hydroxyapatite de calcium (non représentés). En effet, l'hydroxyapatite de calcium est un composant minéral de l'os qui peut être fabriqué chimiquement. L'os voisin identifie l'hydroxyapatite comme un de ses constituants et repousse rapidement sur le revêtement de la prothèse, assurant ainsi une bonne fixation du cotyle dans l'os. Le rôle du titane est d'une part de constituer une barrière à l'hydroxyapatite qui sans cela aurait tendance à diffuser dans le métal constituant le cotyle et d'autre part de créer des micro-reliefs garantissant l'ancrage osseux surfacique. Le revêtement d'hydroxyapatite de calcium peut être obtenu par projection plasma ou par toute autre technique appropriée connue de l'homme de métier. De la même manière le revêtement de titane peut être obtenu par toute technique connue de l'homme de métier, telle que par exemple la projection plasma, ou le dépôt en phase vapeur.

Dans une réalisation fortement préférée du composant cotyloïdien selon l'invention, le revêtement favorisant l'ostéo-intégration, tel qu'un revêtement de titane et d'hydroxyapatite de calcium, est absent sur une couronne **5** de hauteur h sur la périphérie équatoriale **3** du cotyle, afin d'éviter un relargage de matière qui pourrait engendrer une usure éventuelle en formant un troisième corps dans les zones de frottement. La hauteur h est comprise entre 2 et 5 mm, et de préférence comprise entre 3 et 4 mm.

Enfin, la surface interne concave 11 du cotyle selon l'invention est sensiblement hémisphérique et possède un poli de type miroir, obtenu par toute technique connue de l'homme de métier.

Le cotyle selon l'invention peut recevoir soit une tête prothétique de diamètre égal au diamètre interne de la cupule, soit, de préférence, un insert mobile, pouvant être composé de tout matériau apte à limiter le coefficient de frottement de la tête sur le cotyle et résistant à l'usure, ce matériau étant de préférence du polyéthylène. Cet insert mobile est de préférence une cupule de forme sensiblement hémisphérique de diamètre externe égal au diamètre interne de la cupule et de diamètre interne égal à celui de la tête prothétique reçue par ledit insert.

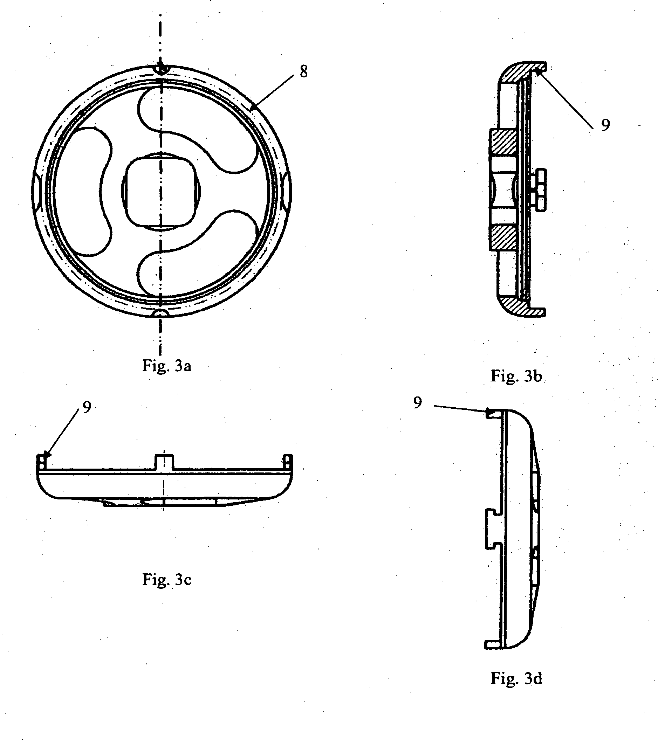

La fixation du cotyle selon l'invention dans l'os iliaque du patient est réalisée à l'aide d'une platine qui permet le vissage et le dévissage du cotyle de manière aisée et précise. La platine est représentée sur la figure 3. En outre, des encoches de vissage et dévissage **4** sont présentes en périphérie du cotyle selon l'invention, telles que représentée sur la figure 1.

La platine permet la préhension et la mise en place du cotyle dans l'os iliaque. Elle comprend une surface **8** de prise d'appui contre le bord du cotyle, elle comprend également des protubérances **9** destinées à s'engager et se bloquer dans les encoches de vissage et de dévissage **4** du cotyle, de manière à permettre une bonne préhension du cotyle par la platine et un serrage par vissage ou un desserrage par dévissage efficace dudit cotyle à l'aide de la platine.

Le cotyle selon l'invention est utilisé de préférence en association avec un élément fémoral comportant de manière habituelle une tige de forme sensiblement rectiligne ancrée dans le fémur et finissant par une extrémité distale conique pour insertion de la tête sphérique de diamètre variable, et une tête sphérique placée à l'extrémité du col prothétique.

## Revendications

1. Composant cotyloïdien de prothèse de hanche, ledit composant cotyloïdien étant creux et en forme de cupule dont la partie externe comporte un filetage pour permettre la fixation dudit composant cotyloïdien dans la cavité cotyloïde de l'os iliaque convenablement préparée, ledit filetage comportant un double filet (20, 21) auto-taraudant interrompu, ledit composant cotyloïdien étant pourvu d'un pôle supérieur effacé (1), et ledit composant cotyloïdien étant pourvu sur sa face externe (10) d'un revêtement favorisant l'ostéo-intégration, et ledit composant cotyloïden étant pourvu d'une surface interne concave sensiblement hémisphérique polie (11), et ledit composant cotyloidien étant **caractérisé en ce que**
- le pas des filetages (20, 21) est décroissant du pôle supérieur (1) vers la périphérie équatoriale (3) du composant cotyloïdien,
- les épaisseurs des filets (20, 21) sont croissantes du pôle supérieur (1) du composant cotyloïdien vers sa périphérie (3),
- l'arête des filets (20, 21) est coupante du côté du pôle (1) du composant cotyloïdien et arrondie ou sensiblement trapézoïdale du côté de la périphérie équatoriale (3) du composant cotyloïdien, le profil de ladite arête variant de façon continue et régulière depuis le pôle (1) vers la périphérie équatoriale (3).

2. Composant cotyloïdien selon la revendication 1, **caractérisé en ce que** ledit revêtement favorisant l'ostéo-intégration est un revêtement de titane poreux associé à un revêtement d'hydroxyapatite de calcium.

3. Composant cotyloïdien selon la revendication 1 ou 2, **caractérisé en ce que** ledit revêtement favorisant l'ostéo-intégration est absent sur une couronne (5) de hauteur h comprise entre 2 et 5 mm sur la périphérie équatoriale (3) du cotyle, et préférentiellement sur une hauteur h comprise entre 3 et 4 mm.

4. Composant cotyloïdien selon l'une quelconque des revendications 1 à 3 **caractérisé en ce qu'**il est réalisé en alliage métallique, et préférentiellement en alliage chrome cobalt ou en acier inoxydable.

5. Composant cotyloïdien selon l'une quelconque des revendications 1 à 4 **caractérisé en ce que** le pas des filets (20, 21) est compris entre 2 et 5 mm, et de préférence entre 3 et 4 mm.

6. Composant cotyloïdien selon l'une quelconque des revendications 1 à 5, **caractérisé en ce que** des encoches de vissage et dévissage (4) sont présentes en périphérie afin de permettre le vissage et le dévissage dudit cotyle dans l'os à l'aide d'une platine.

7. Composant cotyloïdien selon l'une quelconque des revendications 1 à 6 **caractérisé en ce qu'**il comporte en outre un insert mobile en matériau apte à limiter le coefficient de frottement de la tête sur le cotyle et résistant à l'usure, ledit matériau étant de préférence du polyéthylène.

8. Prothèse totale de hanche comprenant un composant cotyloidien selon l'une quelconque des revendications 1 à 7.

## Patentansprüche

1. Gelenkpfannenkomponente von Hüftprothese, wobei die besagte Gelenkpfannenkomponente hohl und in Form einer Schale ist, deren Außenteil ein Gewinde umfasst, um die Fixierung der besagten Gelenkpfannenkomponente in der Hüftgelenkpfanne des angemessen vorbereiteten Hüftknochens zu ermöglichen, wobei das besagte Gewinde einen unterbrochenen, selbstschneidenden doppelten Gewindegang (20, 21) umfasst, die besagte Gelenkpfannenkomponente mit einem zurückgezogenen oberen Pol (1) versehen ist und die besagte Gelenkpfannenkomponente auf ihrer Außenseite (10) mit einer Beschichtung versehen ist, die die Osseointegration begünstigt, und die besagte Gelenkpfannenkomponente mit einer glatten, im Wesentlichen halbkugelförmigen, konkaven Innenfläche (11) versehen ist und die besagte Gelenkpfannenkomponente **dadurch gekennzeichnet ist, dass**
- die Steigung der Gewinde (20, 21) von dem oberen Pol (1) zu dem Äquatorialumfang (3) der Gelenkpfannenkomponente abnimmt,
- die Dicken der Gewindegänge (20, 21) von dem oberen Pol (1) der Gelenkpfannenkomponente zu ihrem Umfang (3) zunehmen,
- die Kante der Gewindegänge (20, 21) auf der Seite des Pols (1) der Gelenkpfannenkomponente schneidend und auf der Seite des Äquatorialumfangs (3) der Gelenkpfannenkomponente abgerundet oder im Wesentlichen trapezförmig ist, wobei das Profil der besagten Kante kontinuierlich und gleichmäßig von dem Pol (1) zu dem Äquatorialumfang (3) variiert.

2. Gelenkpfannenkomponente nach Anspruch 1, **dadurch gekennzeichnet, dass** die besagte Beschichtung, die die Osseointegration begünstigt, eine poröse Titanbeschichtung ist, die mit einer Calciumhydroxyapatit-Beschichtung verbunden ist.

3. Gelenkpfannenkomponente nach Anspruch 1 oder 2, **dadurch gekennzeichnet, dass** die besagte Beschichtung, die die Osseointegration begünstigt, auf einem Kranz (5) mit einer Höhe h, die zwischen 2 und 5 mm liegt, auf dem Äquatorialumfang (3) der Gelenkpfanne und vorzugsweise auf einer Höhe h, die zwischen 3 und 4 mm liegt, fehlt.

4. Gelenkpfannenkomponente nach einem der Ansprüche 1 bis 3, **dadurch gekennzeichnet, dass** sie aus einer Metalllegierung und vorzugsweise aus einer Chrom-KobaltLegierung oder aus Edelstahl hergestellt ist.

5. Gelenkpfannenkomponente nach einem der Ansprüche 1 bis 4, **dadurch gekennzeichnet, dass** die Steigung der Gewinde (20, 21) zwischen 2 und 5 mm und vorzugsweise zwischen 3 und 4 mm liegt.

6. Gelenkpfannenkomponente nach einem der Ansprüche 1 bis 5, **dadurch gekennzeichnet, dass** Einschraub- und Ausschraubkerben (4) am Umfang vorliegen, um das Einschrauben und das Ausschrauben der besagten Gelenkpfanne in bzw. aus dem Knochen mithilfe einer Platte zu ermöglichen.

7. Gelenkpfannenkomponente nach einem der Ansprüche 1 bis 6, **dadurch gekennzeichnet, dass** sie außerdem einen beweglichen Einsatz aus einem Material umfasst, das dazu geeignet ist, den Reibwert des Kopfes auf der Gelenkpfanne zu begrenzen, und das verschleißfest ist, wobei das besagte Material vorzugsweise Polyethylen ist.

8. Hüfttotalprothese, die eine Gelenkpfannenkomponente nach einem der Ansprüche 1 bis 7 umfasst.

## Claims

1. Acetabular component for hip replacement, said acetabular component being hollow and in the shape of a cup wherein the outer portion comprises a thread for enabling the fixation of said acetabular component in the suitably prepared acetabular cavity of the iliac bone, said thread comprising a discontinuous self-tapping double-thread (20, 21), said acetabular component being provided with a receding upper pole (1), and said acetabular component being provided on the outer face (10) thereof with a coating promoting osseointegration, and said acetabular component being provided with a polished substantial hemispherical concave inner surface (11), and said acetabular component being **characterised in that**
- the pitch of the threads (20, 21) is decreasing from the upper pole (1) to the equatorial periphery (3) of the acetabular component,
- the thicknesses of the threads (20, 21) are increasing from the upper pole (1) of the acetabular component towards the periphery (3) thereof,
- the edge of the threads (20, 21) is sharp on the side of the pole (1) of the acetabular component and rounded or substantially trapezoidal on the side of the equatorial periphery (3) of the acetabular component, the profile of said edge varying continuously and regularly from the pole (1) towards the equatorial periphery (3).

2. Acetabular component according to claim 1, **characterised in that** said coating promoting osseointegration is a porous titanium coating associated with a calcium hydroxyapatite coating.

3. Acetabular component according to claim 1 or 2, **characterised in that** said coating promoting osseointegration is absent on a ring (5) of height h between 2 and 5 mm on the equatorial periphery (3) of the acetabulum, and preferentially on a height h between 3 and 4 mm.

4. Acetabular component according to any of claims 1 to 3 **characterised in that** it is made of metal alloy, and preferentially of cobalt-chrome alloy or stainless steel.

5. Acetabular component according to any of claims 1 to 4 **characterised in that** the pitch of the threads (20, 21) is between 2 and 5 mm, and preferably between 3 and 4 mm.

6. Acetabular component according to any of claims 1 to 5, **characterised in that** screwing and unscrewing notches (4) are presented on the periphery so as to enable screwing and unscrewing of said acetabulum in the bone using a plate.

7. Acetabular component according to any of claims 1 to 6 **characterised in that** it further comprises a movable insert made of material suitable for limiting the coefficient of friction of the head on the acetabulum and resistant to wear, said material being preferably polyethylene.

8. Total hip replacement comprising an acetabular component according to any of claims 1 to 7.
